# EUROPEAN PATENT APPLICATION

(11) **EP 3 653 725 A1**
(43) Date of publication of application: **20.05.2020**
(21) Application number: 19198767.6
(22) Date of filing: 23.10.2015
(51) Int. Cl.: C12Q 1/68, C12Q 1/6881

(54) **CELL ASSAY KIT AND METHOD**

(30) Priority: 24.10.2014 GB 201418980
(62) Divisional of application: 15790198.4
(71) Applicant: Fillmore, Helen, Portsmouth Hampshire PO6 3LA (GB); An, Qian, Fareham Hampshire PO14 3AP (GB)
(72) Inventor: Fillmore, Helen, Portsmouth Hampshire PO6 3LA (GB); An, Qian, Fareham Hampshire PO14 3AP (GB)
(74) Representative: Elsy, David

(57) **Abstract**

The application describes a cell line identification assay kit comprising:
(a) one or more genotyping agents;
(b) one or more rodent-specific agents;
(c) one or more mycoplasma-specific markers; and optionally
(d) one or more gender-specific markers.

SEQUENCE LISTING

<110> University of Portsmouth

<120> Cell assay kit and method

<130> P342923PCT

<160> 74

<170> BiSSAP 1.3

<210> 1
<211> 20
<212> DNA
<213> Artificial Sequence

<220>
<223> Primer

<400> 1
ctaaggaatg ggattggtag                                                    20


<210> 2
<211> 20
<212> DNA
<213> Artificial Sequence

<220>
<223> Primer

<400> 2
agacccagtt gatgcaatgt                                                    20


<210> 3
<211> 20
<212> DNA
<213> Artificial Sequence

<220>
<223> Primer

<400> 3
cggctacgct ttaggtgaca                                                    20


<210> 4
<211> 20
<212> DNA
<213> Artificial Sequence

<220>
<223> Primer

<400> 4
tgaaacgggt ggctgtagac                                                    20


<210> 5
<211> 22
<212> DNA

<213> Artificial Sequence

<220>
<223> Primer

<400> 5
tggcttactc acatgattgc tg                                                22


<210> 6
<211> 20
<212> DNA
<213> Artificial Sequence

<220>
<223> Primer

<400> 6
gccccaaaca ctgaaacagg                                                   20


<210> 7
<211> 20
<212> DNA
<213> Artificial Sequence

<220>
<223> Primer

<400> 7
tgctagcagt tggcaagagg                                                   20


<210> 8
<211> 23
<212> DNA
<213> Artificial Sequence

<220>
<223> Primer

<400> 8
aggcctcaac ataggtaccc tta                                               23


<210> 9
<211> 20
<212> DNA
<213> Artificial Sequence

<220>
<223> Primer

<400> 9
   ggccagaatg gggttggtta                                                    20


<210> 10
<211> 21
<212> DNA
<213> Artificial Sequence

<220>

<223> Primer

<400> 10
agagccccac tatggtctac t                                                21


<210> 11
<211> 20
<212> DNA
<213> Artificial Sequence

<220>
<223> Primer

<400> 11
tatgagatgg gcacagcagc                                                  20


<210> 12
<211> 20
<212> DNA
<213> Artificial Sequence

<220>
<223> Primer

<400> 12
cccgggcatc ttgaaatgga                                                  20


<210> 13
<211> 20
<212> DNA
<213> Artificial Sequence

<220>
<223> Primer

<400> 13
ggagtcaaag caggtctcgg                                                  20


<210> 14
<211> 20
<212> DNA
<213> Artificial Sequence

<220>
<223> Primer

<400> 14
tcgagtgtga cagttggctt                                                  20


<210> 15
<211> 20
<212> DNA
<213> Artificial Sequence

<220>
<223> Primer

<400> 15

ttgagaccct tgcacctgac                                                    20


<210> 16
<211> 20
<212> DNA
<213> Artificial Sequence

<220>
<223> Primer

<400> 16
ttgaagacca ccgtgtcctg                                                    20


<210> 17
<211> 20
<212> DNA
<213> Artificial Sequence

<220>
<223> Primer

<400> 17
gagtcaaagc aggtctcgga                                                    20


<210> 18
<211> 20
<212> DNA
<213> Artificial Sequence

<220>
<223> Primer

<400> 18
ttcgagtgtg acagttggct                                                    20


<210> 19
<211> 20
<212> DNA
<213> Artificial Sequence

<220>
<223> Primer

<400> 19
ggcttttggg tcctctgaca                                                    20


<210> 20
<211> 20
<212> DNA
<213> Artificial Sequence

<220>
<223> Primer

<400> 20
tgtggactcc ccatgaaagc                                                    20

<210> 21
<211> 20
<212> DNA
<213> Artificial Sequence

<220>
<223> Primer

<400> 21
tggcacacca cttgtaccac                                                    20


<210> 22
<211> 20
<212> DNA
<213> Artificial Sequence

<220>
<223> Primer

<400> 22
taagccacct acttgccagc                                                    20


<210> 23
<211> 20
<212> DNA
<213> Artificial Sequence

<220>
<223> Primer

<400> 23
aggacgggca agcttttcat                                                    20


<210> 24
<211> 20
<212> DNA
<213> Artificial Sequence

<220>
<223> Primer

<400> 24
aggcttcccc tctgtaccat                                                    20


<210> 25
<211> 20
<212> DNA
<213> Artificial Sequence

<220>
<223> Primer

<400> 25
gcatcgtaat cagctgcgtc                                                    20


<210> 26
<211> 20
<212> DNA

<213> Artificial Sequence

<220>
<223> Primer

<400> 26
actaagatgc agcaggtggg                                                    20


<210> 27
<211> 20
<212> DNA
<213> Artificial Sequence

<220>
<223> Primer

<400> 27
ggcctccact ctgtctgttc                                                    20


<210> 28
<211> 20
<212> DNA
<213> Artificial Sequence

<220>
<223> Primer

<400> 28
tccacaggca ctgtcaacaa                                                    20


<210> 29
<211> 20
<212> DNA
<213> Artificial Sequence

<220>
<223> Primer

<400> 29
gggaggaacc atggaactcg                                                    20


<210> 30
<211> 20
<212> DNA
<213> Artificial Sequence

<220>
<223> Primer

<400> 30
aagtcgactg gtacgttgct                                                    20


<210> 31
<211> 20
<212> DNA
<213> Artificial Sequence

<220>

<223> Primer

<400> 31
gggtgtaggt cttccatgcc                                                    20


<210> 32
<211> 20
<212> DNA
<213> Artificial Sequence

<220>
<223> Primer

<400> 32
ctcgcggtaa ctcttccgag                                                    20


<210> 33
<211> 19
<212> DNA
<213> Artificial Sequence

<220>
<223> Primer

<400> 33
actgcagtcc aatctgggt                                                     19


<210> 34
<211> 21
<212> DNA
<213> Artificial Sequence

<220>
<223> Primer

<400> 34
atgaaatcaa cagaggcttg c                                                  21


<210> 35
<211> 23
<212> DNA
<213> Artificial Sequence

<220>
<223> Primer

<400> 35
ggtgattttc ctctttggta tcc                                                23


<210> 36
<211> 26
<212> DNA
<213> Artificial Sequence

<220>
<223> Primer

<400> 36

agccacagtt tacaacattt gtatct                                          26


<210> 37
<211> 21
<212> DNA
<213> Artificial Sequence

<220>
<223> Primer

<400> 37
atgttggtca ggctgactat g                                               21


<210> 38
<211> 24
<212> DNA
<213> Artificial Sequence

<220>
<223> Primer

<400> 38
gattccacat ttatcctcat tgac                                            24


<210> 39
<211> 32
<212> DNA
<213> Artificial Sequence

<220>
<223> Primer

<400> 39
attgcaactt atatgtattt ttgtatttca tg                                    32


<210> 40
<211> 28
<212> DNA
<213> Artificial Sequence

<220>
<223> Primer

<400> 40
accaaattgt gttcatgagt atagtttc                                        28


<210> 41
<211> 26
<212> DNA
<213> Artificial Sequence

<220>
<223> Primer

<400> 41
atcacagaag tctgggatgt ggagga                                          26

<210> 42
<211> 19
<212> DNA
<213> Artificial Sequence

<220>
<223> Primer

<400> 42
ggcagcccaa aaagacaga                                                    19


<210> 43
<211> 24
<212> DNA
<213> Artificial Sequence

<220>
<223> Primer

<400> 43
gggggtctaa gagcttgtaa aaag                                              24


<210> 44
<211> 29
<212> DNA
<213> Artificial Sequence

<220>
<223> Primer

<400> 44
gtttgtgtgt gcatctgtaa gcatgtatc                                         29


<210> 45
<211> 20
<212> DNA
<213> Artificial Sequence

<220>
<223> Primer

<400> 45
ttcttgagcc cagaaggtta                                                   20


<210> 46
<211> 27
<212> DNA
<213> Artificial Sequence

<220>
<223> Primer

<400> 46
attctaccag caacaacaca aataaac                                           27


<210> 47
<211> 22
<212> DNA

<213> Artificial Sequence

<220>
<223> Primer

<400> 47
atatgtgagt caattcccca ag                                                22


<210> 48
<211> 26
<212> DNA
<213> Artificial Sequence

<220>
<223> Primer

<400> 48
tgtattagtc aatgttctcc agagac                                            26


<210> 49
<211> 24
<212> DNA
<213> Artificial Sequence

<220>
<223> Primer

<400> 49
ccggaggtaa aggtgtctta aagt                                              24


<210> 50
<211> 22
<212> DNA
<213> Artificial Sequence

<220>
<223> Primer

<400> 50
atttcctgtg tcagaccctg tt                                                22


<210> 51
<211> 20
<212> DNA
<213> Artificial Sequence

<220>
<223> Primer

<400> 51
ggctgcaggg cataacatta                                                   20


<210> 52
<211> 24
<212> DNA
<213> Artificial Sequence

<220>

<223> Primer

<400> 52
attctatgac tttgcgcttc agga                                              24


<210> 53
<211> 19
<212> DNA
<213> Artificial Sequence

<220>
<223> Primer

<400> 53
gaaggtcgaa gctgaagtg                                                    19


<210> 54
<211> 27
<212> DNA
<213> Artificial Sequence

<220>
<223> Primer

<400> 54
attagaattc tttaatctgg acacaag                                           27


<210> 55
<211> 26
<212> DNA
<213> Artificial Sequence

<220>
<223> Primer

<400> 55
attaccaaca tgaaagggta ccaata                                            26


<210> 56
<211> 33
<212> DNA
<213> Artificial Sequence

<220>
<223> Primer

<400> 56
tgggttatta attgagaaaa ctccttacaa ttt                                    33


<210> 57
<211> 21
<212> DNA
<213> Artificial Sequence

<220>
<223> Primer

<400> 57

gtgattccca ttggcctgtt c                                                21

<210> 58
<211> 22
<212> DNA
<213> Artificial Sequence

<220>
<223> Primer

<400> 58
cctcctgtgg gctgaaaagc tc                                               22

<210> 59
<211> 20
<212> DNA
<213> Artificial Sequence

<220>
<223> Primer

<400> 59
gcacagaaca ggcacttagg                                                  20

<210> 60
<211> 18
<212> DNA
<213> Artificial Sequence

<220>
<223> Primer

<400> 60
cgctcaaacg tgaggttg                                                    18

<210> 61
<211> 33
<212> DNA
<213> Artificial Sequence

<220>
<223> Primer

<400> 61
gccctagtgg atgataagaa taatcagtat gtg                                    33

<210> 62
<211> 30
<212> DNA
<213> Artificial Sequence

<220>
<223> Primer

<400> 62
ggacagatga taaatacata ggatggatgg                                       30

<210> 63
<211> 24
<212> DNA
<213> Artificial Sequence

<220>
<223> Primer

<400> 63
ccctgggctc tgtaaagaat agtg                                              24


<210> 64
<211> 24
<212> DNA
<213> Artificial Sequence

<220>
<223> Primer

<400> 64
atcagagctt aaactgggaa gctg                                              24


<210> 65
<211> 20
<212> DNA
<213> Artificial Sequence

<220>
<223> Primer

<400> 65
aggccattct tcattctcgg                                                   20


<210> 66
<211> 21
<212> DNA
<213> Artificial Sequence

<220>
<223> Primer

<400> 66
tggggaaata aagtggacct g                                                 21


<210> 67
<211> 19
<212> DNA
<213> Artificial Sequence

<220>
<223> Primer

<400> 67
ggtctcctct acctctgtc                                                    19


<210> 68
<211> 20
<212> DNA

<213> Artificial Sequence

<220>
<223> Primer

<400> 68
ccaagtttgc aaagggcaag                                                    20


<210> 69
<211> 25
<212> DNA
<213> Artificial Sequence

<220>
<223> Primer

<400> 69
cagacgttaa cataatctga gaggg                                              25


<210> 70
<211> 22
<212> DNA
<213> Artificial Sequence

<220>
<223> Primer

<400> 70
gtagagctgg accaactatc ca                                                 22


<210> 71
<211> 19
<212> DNA
<213> Artificial Sequence

<220>
<223> Primer

<400> 71
gggttgcgct cgttgcagg                                                     19


<210> 72
<211> 20
<212> DNA
<213> Artificial Sequence

<220>
<223> Primer

<400> 72
cagatggtgc atggttgtcg                                                    20


<210> 73
<211> 20
<212> DNA
<213> Artificial Sequence

<220>

<223> Primer

<400> 73
gttactcacc cattcgccgc                                                    20


<210> 74
<211> 20
<212> DNA
<213> Artificial Sequence

<220>
<223> Primer

<400> 74
gctggctgtg tgcctaatac                                                    20

## Description

The invention provides an assay kit and method to allow the detection of genotype markers in a human cell line and additionally determines if the cell line is contaminated with rodent DNA or mycoplasma.

The importance of cell line identification has been well recognised by the scientific community and recently a list of 360 cross-contaminated and misidentified cell lines has been published, and is being updated when necessary (1). The urgent need for routine validation tests when using cultured human cells has prompted the internationally accepted proposal of using short tandem repeat (STR) DNA genotyping for cell identification test (2-4). STR loci are DNA fragments consisting of tandem repetitive sequence elements usually of 3-7 base pairs (bp) in length. These STR loci are well distributed throughout the human genome and utilised as highly polymorphic markers, which can be detected after amplification by polymerase chain reaction (PCR). The standard methodology of STR genotyping consists of two steps; the initial co-amplification of a panel of STR markers by multiplex PCR followed by the downstream fragment length analysis (FLA). After PCR, alleles of STR markers can be differentiated by different fragment sizes due to variable copy numbers of the repeat elements contained within the amplified regions. The American Type Culture Collection (ATCC) Standards Development Organization Workgroup has issued standard ASN-0002, which recommends using a panel of at least 8 highly informative STR loci (i.e. D5S818, D7S820, D13S317, D16S539, CSF1PO, TH01, TPOX and vWA) plus Amelogenin for gender identification, for human cell line identification (5, 6). These ASN-0002 markers are included in the commonly used commercial STR-PCR kits, GenePrint® 10 System and PowerPlex® 16 HS System (Promega) (7, 8), as well as in the preferred "3-in-1" systems under this invention.

At present the standardised technology for FLA is capillary electrophoresis which separates and detects the fluorescence-tagged STR fragments (2-4). There are a number of commercially available STR-PCR kits commonly used that provide convenient multiplex PCR amplification of the markers (9). The downstream FLA by capillary electrophoresis, however, is time-consuming and costly because it requires state-of-the-art facilities and properly trained specialists to analyse the data obtained, resulting in outsourcing of the cell identification service for the majority of research laboratories.

In a recent study designed and performed by the Applicants, the potential of other electrophoresis platforms for FLA, e.g. the Agilent 2100 Bioanalyzer, was explored. The Bioanalyzer is a microfluidics-based electrophoresis system for sizing and quantification of DNA, RNA, protein and cells (10-13). It is also used for quality check of DNA, RNA and protein samples in a broad range of molecular assays (14-16). Recently the Bioanalyzer has been employed to resolve STR fragments in forensic samples as well as to identify fish species based on their restriction fragment length polymorphism pattern (17, 18). The Applicant's study showed that a commercial STR-PCR kit, the StemElite ID System from Promega which has now been replaced by the GenePrint® 10 System consisting of the same STR markers (7), was valid for use with a microfluidic electrophoresis system, i.e. the Bioanalyzer (19). This novel FLA method demonstrated excellent accuracy and reproducibility in our hands and the STR profiles of 10 human cell lines obtained from the Bioanalyzer method were highly comparable to those from the conventional method, i.e. capillary electrophoresis (19). In conclusion, the Bioanalyzer has proved to be a more user-friendly and cost-effective alternative to the standard capillary electrophoresis for the STR-based cell identification test. Furthermore, as many research laboratories have access to the Bioanalyzers in core facilities for other applications such as DNA/RNA/protein quality and quantity assessment, we believe that this new method for cell line identification is highly valuable and beneficial for cell culture laboratories (19).

In addition to the risk of cross-contamination amongst human cell lines, mycoplasma contamination presents a serious concern in cell culture as it affects the biological behaviours of the infected cells and invalidates research findings. Mycoplasmas are one of the smallest free-living forms of bacteria that widely spread in nature (20). Unique features of mycoplasmas include the absence of a cell wall and their flexible membrane, both of which result in differing shapes of those microbes and cause difficulties in their detection even under electron microscopes (reviewed in Nikfarjam et al., 2012) (21). It is estimated that the mycoplasma contamination rate in cultured human cells is about 15-35% in the United States and European countries (22, 23). There are several common sources for mycoplasma contamination in cell culture associated with human, bovine and swine species, including personnel in the cell culture laboratories, foetal bovine serum and trypsin solutions derived from swines (24, 25). Currently there is a range of commercial mycoplasma detection kits available based mainly on enzymatic or PCR assays (reviewed in Volokhov et al., 2011) (26). Apart from mycoplasma infection, another major issue in human cell culture is the risk of rodent cell contamination, since mouse and rat cells are widely cultured in research laboratories. Additionally, as there has been a significant increase in the use of human cancer cells being cultured and maintained in rodent xenograft models, this kit will be extremely valuable for assuring there is no cross-contamination in human cells with rodent DNA. Therefore it will be an added value to include mouse/rat markers in the human cell identification test system. This invention aims to develop two "3-in-1" kits (see details below) for the co-amplification of human-specific STR alongside mouse/rat and mycoplasma markers. In addition the "3-in-1" systems may include, for example, 2 extra human gender-determining markers apart from the standard marker, Amelogenin, which tends to be lost on chromosome Y in cancer tissues due to genetic instability causing inaccurate gender profile of the tissue and tissue-derived cell line (27).

This invention simplifies, expands, and improves current commercial methodologies in two basic ways. The combination of identifying cell lines and at the same time testing for mycoplasma and rodent cross-contamination is innovative and timely as the need for authentication and validation of cell lines is increasing. Currently cell identification, mycoplasma contamination, and rodent cross-contamination are assessed separately by different methods and this invention combines these assays into 1 ("3-in-1"). In addition, by adding novel human, mycoplasma, and rodent markers and optimising the components of this "3-in-1" kit for analysis using microfluidic systems, this method can be widely used at a significant time and cost reduction compared to the current single assay methods.

The invention provides a cell line identification assay kit comprising:
(a) one or more genotyping agents;
(b) one or more rodent-specific agents;
(c) one or more mycoplasma-specific agents; and optionally
(d) one or more gender-specific agents.

The agents are each capable of identifying a specific marker, such as a genotyping marker, rodent-specific marker, mycoplasma-specific marker or gender-specific marker. Typically the cell line identification assay kit is adapted to use polymerase chain reaction (PCR) to identify the presence of the markers in the cell line. The agents are therefore typically a primer or a pair of primers comprising a polynucleotide sequence which is specific for a genotyping marker, rodent-specific marker, mycoplasma-specific marker or gender-specific marker.

Typically the cell line is a human cell line. The cell line is typically a culture of cells grown *in vitro.* It may be, for example, a stem cell line, such as a pluripotent or totipotent cell line, a cancer cell line or a tissue cell line.

In addition, this assay may be particularly useful to provide an accurate gender determination for male-derived cancer cell lines which have lost the Amelogenin marker on chromosome Y due to genetic instability and consequently yield the X/X female readout using the existing commercial kits.

The assay is also useful to test human cell lines which have been cultured in a laboratory where rodent cells are also cultured or where human cancer cells have been directly passaged and maintained using a rodent xenograft model.

The assay kit is typically adapted to be used with the agents mixed together with a single DNA sample from the cell line. That is the agents may be mixed together in a single container or a "one-pot" system, with at least a portion of that mixture incubated with DNA from said cell line, being analysed and the presence or absence of the markers being determined from that same sample.

The agents may therefore be provided mixed together.

The ability to detect the markers in a single DNA sample at the same time means that the user can readily identify whether the results of the genotyping are correct and that, at that time, the cell line was free of rodent-specific markers and mycoplasma-specific markers. This considerably improves upon the state-of-the-art methodology and in turn the confidence in any results obtained from using the cell line.

Typically each agent is capable of producing a different detectable product. For example, in a PCR based system the primers are used, in combination with a suitable polymerase, buffer and deoxynucleotide triphosphate (dNTP), together with thermal cycling, to produce an identifiable product. That product may be selected so that each agent is capable of producing a different detectable product of a different size. For example, the size of the product of the rodent-specific agent and the mycoplasma-specific agent may be selected to not substantially overlap with the size of the product of the other genotyping agents. The detectable product of the rodent agents and the mycoplasma agents may be polynucleotides and may, for example, be larger or smaller than the products of the genotyping agents. They may have a size larger than 500bp (or may be of 500bp) or have a size of less than 100bp as to not interfere with human markers (100-474bp).

Processes for genotyping cell lines, for example, using the detection of polymorphisms in DNA is described in US re-issued patent RE 37,984, and as a multiplex system in US 5,843,660. In such systems, and indeed in a preferred aspect of the current invention, at least one part of the region to be analysed in a sample is annealed with one of the molecules of a primer pair that is substantially complementary to one of the 5' or 3' flanks of a marker sequence, such as a short terminal repeat (STR) region. The annealing occurs in such an orientation that the synthesis products obtained by a primer-controlled polymerisation reaction with one of the primers, can serve as a template for annealing the other primer after denaturation. Primer-controlled polymerase chain reaction is carried out and the products of that reaction are separated and analysed.

There are a number of methods generally known in the art for the detection of the products of multiplex PCR reactions. Many of them separate the products of the polymerase chain reaction from each other using electrophoresis. Typically the electrophoresis is capillary electrophoresis or microfluidic electrophoresis. The products may be labelled with, for example, a fluorescent marker to allow the detection of the product, for example, after it has passed through a capillary electrophoresis system. Alternatively, the product need not be labelled, but may still be detected using, for example, microfluidic systems. Microfluidic systems are typically used as they have the advantages described above. The kit may be a microfluidic kit, for example adapted to be used with microfluidic electrophoresis.

Typically the markers detected by the genotyping agents are short tandem repeat (STR) loci. As described above, the ATCC has issued standard ASN-0002. This recommends using a panel of at least 8 informative STR loci. The assay kit typically comprises the following STR markers:
D5S818, D7S820, D13S317, D16S539, CSF1PO, TH01, TPOX, and vWA.

The STR markers may additionally comprise any number, one, two, three or indeed all of the following markers selected from:
D21S11, D3S1385, D8S1179, D18S51, FGA, Penta D and Penta E

### Locus Specific Information for Markers

From PowerPlex™ 16 HS System Manual, Promega Inc

| **STR Locus** | **Label** | **Size Range of Allelic Ladder Components^{1,2} (bases)** | **Repeat Numbers of Allelic Ladder Components²** |
|---|---|---|---|
| Penta E | FL | 379-474 | 5-24 |
| D18S51 | FL | 290-366 | 8-10,10.2,11-13,13.2,14-27 |
| D21S11 | FL | 203-259 | 24, 24.2, 25, 25.2, 26-28, 28.2, 29, 29.2, 30, 30.2, 31, 31.2, 32, 32.2, 33, 33.2, 34 |
| TH01 | FL | 156-195 | 4-9, 9.3, 10-11, |
| D3S135 | FL | 115-147 | 13.3 12-20 |
| FGA | TMR | 322-444 | 16-18, 18.2, 19, 19.2, 20, 20.2, 21, 21.2, 22, 22.2, 23, 23.2, 24, 24.2, 25, 25.2, |
| TPOX | TMR | 262-290 | 6-13 |
| D8S1179 | TMR | 203-247 | 7-18 |
| vWA | TMR | 123-171 | 10-22 |
| Amelogenin | TMR | 106, 112 | X, Y 2.2, 3.2, |
| Penta D | JOE | 376-449 | 5,7-17 |
| CSF1PO | JOE | 321-357 | 6-15 5, |
| D16S53 | JOE | 264-304 | 8-15 |
| D7S820 | JOE | 215-247 | 6-14 |
| D13S317 | JOE | 176-208 | 7-15 |
| D5S818 | JOE | 119-155 | 7-16 |

| | | | |
|---|---|---|---|
| ¹The length of each allele in the allelic ladder has been confirmed by sequence analyses. ²For a current list of microvariants, see the Variant Allele Report published at the U.S. National Institute of Standards and Technology (NIST) web site at: www.cstl.nist.gov/div831/strbase/ | | | |

The assay kit may comprise two or more mycoplasma-specific agents. Using two or more different mycoplasma-specific agents increases the ability of the kit to detect a broader range of mycoplasma contaminants. For example, two mycoplasma agents may be used. Typically these may be used to detect a 70bp and a 1062bp mycoplasma-specific sequences of the 16S ribosomal RNA (rRNA) gene to ensure accurate detection and prevent false positives. Typically the mycoplasma markers are able to detect one or more or all of the following mycoplasma contaminants: *Mycoplasma arginini*, *Mycoplasma fermentans*, *Mycoplasma hominis*, *Mycoplasma hyorhinis*, *Mycoplasma pirum*, *Mycoplasma orale*, *Mycoplasma salivarium* and *Acholeplasma laidlawii.*

Typically the rodent-specific agents comprise a rat-specific agent and one or more, typically two mouse-specific agents, which are capable of detecting rat-specific markers and mouse-specific markers. The rodent-specific agent may also be, for example, hamster, guinea pig or squirrel-specific. The rodent-specific agent may be a mouse-specific agent which binds to the major urinary protein isoform X1 gene on chromosome 4 (NCBI Genbank accession No. NC_000070.6). The rodent-specific agent may be a rat-specific agent which binds to the GTP-binding protein Rheb precursor on chromosome 4 (NCBI Genbank accession No. AC_000072.1).

The optional gender-specific agent may, for example, be an Amelogenin agent such as a primer for Amelogenin. Amelogenin is generally known in the art for gender identification, and indeed is one of the markers recommended by standard ASN-0002. The problem of Amelogenin is that it tends to be lost on chromosome Y in cancer tissues, due to genetic instability, causing inaccurate gender profile of the tissue and tissue-derived cell line. Accordingly, the assay kit may comprise two or more Y chromosome-specific agents. The Y chromosome-specific agents may be selected so that they are distanced from each other on the Y chromosome, therefore improving the chance that at least one of the markers will still be present if there is genetic instability within the cell line. For example, one of the gender-specific agents may be specific for a part of the region of 0-4Mb, 12-18Mb or 20-28Mb of the Y chromosome. One may be Amelogenin or both may be different to Amelogenin. Alternatively, three or more agents may be used, for example including Amelogenin. The marker may, for example, be DYS392. There is a commercially available test for this marker. The marker may be modified, for example, to locate at 3bp distance from the 3' end of the Y-specific STR marker DYS392. The marker DYS392-UoP, as described below, contains a 112bp chromosome-Y-specific sequence. DYS392 has been reported to be intact in cancer tissues with missing Amelogenin marker (27). Therefore DYS392-UoP may provide a more accurate and reliable marker for chromosome Y detection, compared with Amelogenin. The Y-specific markers may also be selected from the following: UoP-Y1-15 (Table 1). Initial data suggests that UoP-Y2, 3, 6, 8, 14 and 15 are especially useful as gender markers and may therefore be preferred.

Where the specific agents are, for example, primers, the primers may be adapted to ensure that they have similar melting temperatures when annealed to a target DNA sequence. This allows, for example, the primers to be used within a one-pot system with the primers mixed together within the same sample of the cell line. Alternatively, or additionally, the buffer used with the system may be adapted to optimise the production of products from the primers.

The primers may be selected from those listed in Tables 1-3.

**Table 1. Sequences of PCR primers and sizes of PCR products of the novel Y-specific markers designed by the invention**

| Primers | Sequences | Product Sizes (bp) |
|---|---|---|
| DYS392-UoP-F | CTAAGGAATGGGATTGGTAG | 112 |
| DYS392-UoP-R | AGACCCAGTTGATGCAATGT | |
| UoP-Y1-F | CGGCTACGCTTTAGGTGACA | 112 |
| UoP-Y1-R | TGAAACGGGTGGCTGTAGAC | |
| UoP-Y2-F | TGGCTTACTCACATGATTGCTG | 112 |
| UoP-Y2-R | GCCCCAAACACTGAAACAGG | |
| UoP-Y3-F | TGCTAGCAGTTGGCAAGAGG | 112 |
| UoP-Y3-R | AGGCCTCAACATAGGTACCCTTA | |
| UoP-Y4-F | GGCCAGAATGGGGTTGGTTA | 112 |
| UoP-Y4-R | AGAGCCCCACTATGGTCTACT | |
| UoP-Y5-F | TATGAGATGGGCACAGCAGC | 112 |
| UoP-Y5-R | CCCGGGCATCTTGAAATGGA | |
| UoP-Y6-F | GGAGTCAAAGCAGGTCTCGG | 112 |
| UoP-Y6-R | TCGAGTGTGACAGTTGGCTT | |
| UoP-Y7-F | TTGAGACCCTTGCACCTGAC | 112 |
| UoP-Y7-R | TTGAAGACCACCGTGTCCTG | |
| UoP-Y8-F | GAGTCAAAGCAGGTCTCGGA | 112 |
| UoP-Y8-R | TTCGAGTGTGACAGTTGGCT | |
| UoP-Y9-F | GGCTTTTGGGTCCTCTGACA | 112 |
| UoP-Y9-R | TGTGGACTCCCCATGAAAGC | |
| UoP-Y10-F | TGGCACACCACTTGTACCAC | 112 |
| UoP-Y10-R | TAAGCCACCTACTTGCCAGC | |
| UoP-Y11-F | AGGACGGGCAAGCTTTTCAT | 112 |
| UoP-Y11-R | AGGCTTCCCCTCTGTACCAT | |
| UoP-Y12-F | GCATCGTAATCAGCTGCGTC | 112 |
| UoP-Y12-R | ACTAAGATGCAGCAGGTGGG | |
| UoP-Y13-F | GGCCTCCACTCTGTCTGTTC | 112 |
| UoP-Y13-R | TCCACAGGCACTGTCAACAA | |
| UoP-Y14-F | GGGAGGAACCATGGAACTCG | 112 |
| UoP-Y14-R | AAGTCGACTGGTACGTTGCT | |
| UoP-Y15-F | GGGTGTAGGTCTTCCATGCC | 112 |
| UoP-Y15-R | CTCGCGGTAACTCTTCCGAG | |

**Table 2. Sequences of PCR primers and sizes of PCR products of the human STR markers**

| Primers | Sequences | Product Sizes (bp) |
|---|---|---|
| D3S1358*-F | ACTGCAGTCCAATCTGGGT | 115-147 |
| D3S1358*-R | ATGAAATCAACAGAGGCTTGC | |
| D5S818-F | GGTGATTTTCCTCTTTGGTATCC | 119-155 |
| D5S818-R | AGCCACAGTTTACAACATTTGTATCT | |
| D7S820-F | ATGTTGGTCAGGCTGACTATG | 215-247 |
| D7S820-R | GATTCCACATTTATCCTCATTGAC | |
| D8S1179*-F | ATTGCAACTTATATGTATTTTTGTATTTCATG | 203-247 |
| D8S 1179*-R | ACCAAATTGTGTTCATGAGTATAGTTTC | |
| D13S317-F^{#} | ATCACAGAAGTCTGGGATGTGGAGGA | 176-208 |
| D13S317-R | GGCAGCCCAAAAAGACAGA | |
| D16S539-F | GGGGGTCTAAGAGCTTGTAAAAAG | 264-304 |
| D16S539-R | GTTTGTGTGTGCATCTGTAAGCATGTATC | |
| D18S51*-F | TTCTTGAGCCCAGAAGGTTA | 290-366 |
| D18S51*-R | ATTCTACCAGCAACAACACAAATAAAC | |
| D21S11-F | ATATGTGAGTCAATTCCCCAAG | 203-259 |
| D21S11-R | TGTATTAGTCAATGTTCTCCAGAGAC | |
| CSF1PO-F | CCGGAGGTAAAGGTGTCTTAAAGT | 321-357 |
| CSF1PO-R | ATTTCCTGTGTCAGACCCTGTT | |
| FGA*-F | GGCTGCAGGGCA T A AC ATT A | 322-444 |
| FGA*-R | ATTCTATGACTTTGCGCTTCAGGA | |
| Penta D*-F | GAAGGTCGAAGCTGAAGTG | 376-449 |
| Penta D*-R | ATTAGAATTCTTTAATCTGGACACAAG | |
| Penta E*-F | ATTACCAACATGAAAGGGTACCAATA | 379-474 |
| Penta E*-R | TGGGTTATTAATTGAGAAAACTCCTTACAATTT | |
| TH01-F | GTGATTCCCATTGGCCTGTTC | 156-195 |
| TH01-R^{#} | CCTCCTGTGGGCTGAAAAGCTC | |
| TPOX-F | GCACAGAACAGGCACTTAGG | 262-290 |
| TPOX-R | CGCTCAAACGTGAGGTTG | |
| vWA-F | GCCCTAGTGGATGATAAGAATAATCAGTATGTG | 123-171 |
| vWA-R | GGACAGATGATAAATACATAGGATGGATGG | |
| Amelogenin-F^{#} | CCCTGGGCTCTGTAAAGAATAGTG | 106,112 |
| Amelogenin-R | ATCAGAGCTTAAACTGGGAAGCTG | |

| | | |
|---|---|---|
| * Extra STR markers included in the larger-panel system. ^{#} Modified primer sequence based on published data (Ref. 28), i.e. with modified or extra nucleotides (underlined) for PCR optimisation purpose. | | |

**Table 3. Sequences of PCR primers and sizes of PCR products of the novel rodent/mycoplasma markers designed by the invention**

| Primers | Sequences | Product Sizes (bp) |
|---|---|---|
| Mouse-Fl | AGGCCATTCTTCATTCTCGG | 90 |
| Mouse-R1 | TGGGGAAATAAAGTGGACCTG | |
| Mouse-F2 | GGTCTCCTCTACCTCTGTC | 90 |
| Mouse-R2 | CCAAGTTTGCAAAGGGCAAG | |
| Rat-F | CAGACGTTAACATAATCTGAGAGGG | 500 |
| Rat-R | GTAGAGCTGGACCAACTATCCA | |
| Mycoplasma-F1* | GGGTTGCGCTCGTTGCAGG | 70 |
| Mycoplasma-R1 | CAGATGGTGCATGGTTGTCG | |
| Mycoplasma-F2 | GTTACTCACCCATTCGCCGC | 70 |
| Mycoplasma-R2* | GCTGGCTGTGTGCCTAATAC | |

| | | |
|---|---|---|
| * The mycoplasma primers, mycoplasma-F1 and R2, also amplify a second mycoplasma-specific sequence of 1062bp on the 16s rRNA gene. | | |

The invention also provides a primer having a sequence as shown in Tables 1-3. The primer may be the Amelogenin-F primer having the sequence in Table 1, or the mouse, rat, mycoplasma, DYS392-UoP, D13S317-F, or TH01-R primers in Tables 1-3.

Typically, the kit contains PCR primers, a polymerase or a fragment of a polymerase along with deoxy nucleotide triphosphates to allow PCR to be carried out on the sample. A polymerase may, for example, be GoTaq™ G2 Hot Start polymerase which is available from Promega. The kit may also comprise a buffer containing, for example, magnesium chloride.

The kit may also contain PCR primers labelled with a dye or a fluorescent molecule. Products may also be detected by, for example, capillary electrophoresis.

Additionally, the kit may comprise a rodent-specific control and a mycoplasma-specific control. For example, the controls may be a portion of rodent-specific polynucleotide sequence and a portion of a mycoplasma-specific polynucleotide sequence, to which the specific agents such as primer pairs bind. These may be used as controls to ensure that the kit is working correctly.

A further aspect of the invention provides a method of assaying a cell line comprising providing a sample of cell line and testing the sample for the presence of:
(a) one or more genotyping markers;
(b) one or more rodent-specific markers;
(c) one or more mycoplasma-specific markers; and optionally
(d) one or more gender-specific markers.

Typically each of the markers is tested for within the same sample from the cell line, and a single portion of the sample may be analysed for the microbes. The cell line may be tested using, for example, a kit according to the invention. Typically cells are collected, DNA extracted from the cells, and the agents for detecting the markers may then be added. The method may comprise the use of marker-specific primers as the agents to detect the specific markers, the product of a PCR reaction being detected by, for example, fragment length analysis using, for example, electrophoresis, such as capillary electrophoresis or microfluidic electrophoresis.

The invention also provides a machine readable medium, storing executable instructions that when executed by a data processing system cause the system to perform a method comprising:
(i) obtaining data values obtained by testing a cell line for the presence of:
   (a) one or more genotyping markers;
   (b) one or more rodent-specific markers;
   (c) one or more mycoplasma-specific markers; and optionally
   (d) one or more gender specific markers;
(ii) comparing the data to predetermined values corresponding to the presence of each marker in a cell line such as allele numbers of the markers of a known commercial cell line; and
(iii) providing a readout showing the presence or absence of each marker in the cell line.

The data values may, for example, be the position of peaks or bands of products of PCR reactions. The peaks or bands, correspond to the presence or indeed absence of the markers in the cell. The data values may be from a simple source, such as from a single sample which is analysed, for example, to produce a series of peaks or bands.

The predetermined values may, for example, be the positions of expected peaks, corresponding to the expected markers if they are present within the cell line. The method typically uses data values which are obtained by methods of the invention or indeed kit of the invention.

A further aspect of the invention provides capillary electrophoresis apparatus or microfluidic electrophoresis apparatus comprising a machine readable medium according to the invention and/or an assay kit according to the invention.

Typically the machine readable medium is a non-transitory medium or a storage medium, especially a non-transitory storage medium.

The invention will now be described by way of examples only with reference to the following figures.
Figure 1: STR profile of SNB-19 cell line. STR-PCR was performed using the Promega GenePrint® 10 system. Fragment length analysis was carried out using a microfluidic electrophoresis instrument, the Agilent 2100 Bioanalyzer.
Figure 2: Overlaid profiles of five DNA genotyping samples from different passages of the same cell line, UP-029.
Figure 3: Comparison of STR profiles of two cell lines, UP-007 and UP-019.

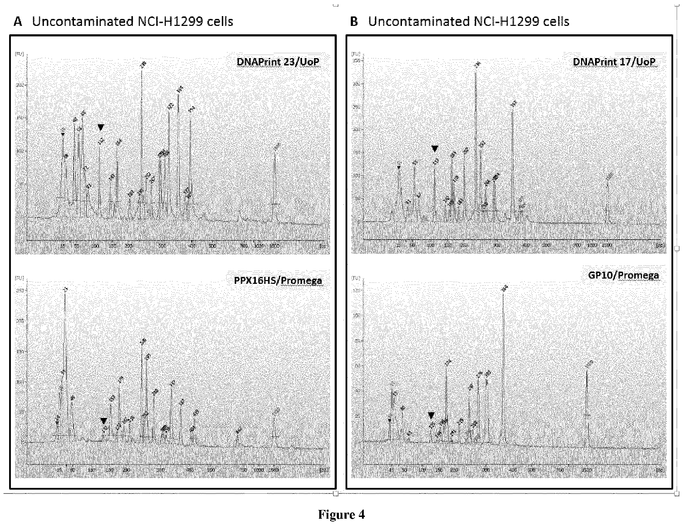

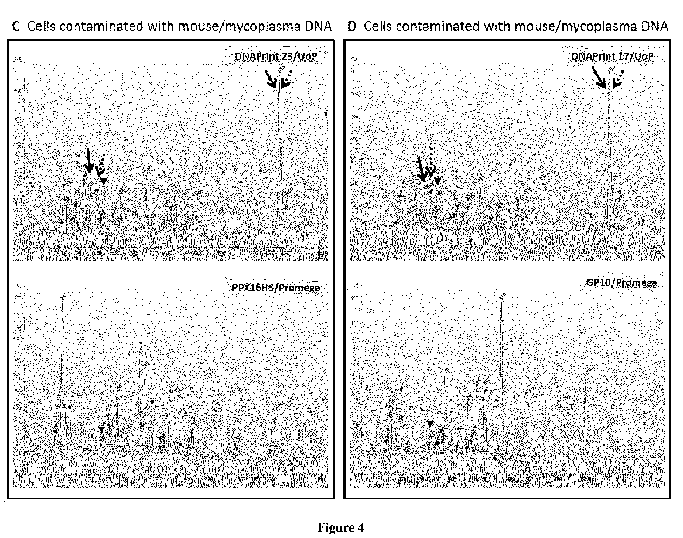

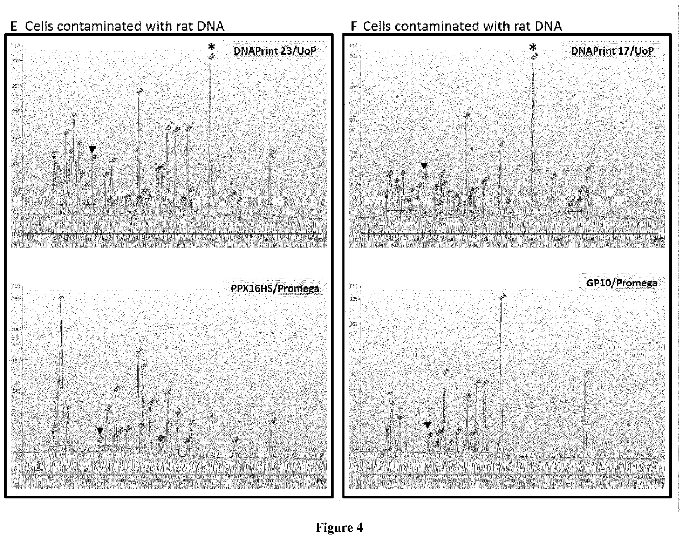

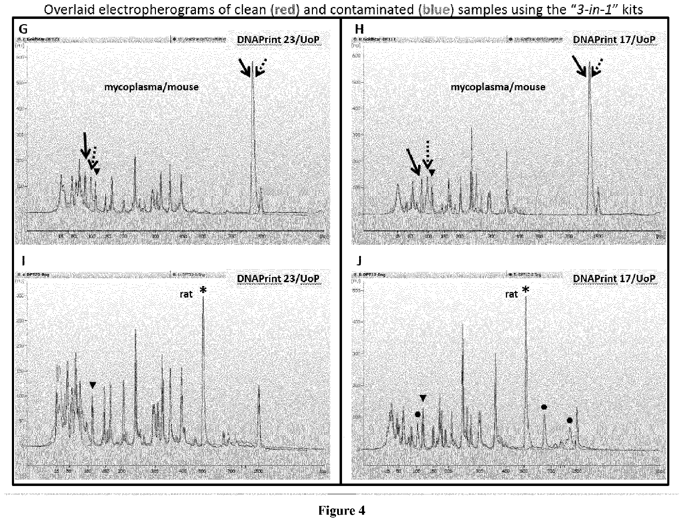

Figure 4: STR profiles of NCI-H1299 using the "3-in-1" DNAPrint 23 and DNAPrint 17 kits (A-F; top panels), as compared to the profiles using the commercial PowerPlex® 16 HS (PPX16HS) and GenePrint® 10 (GP10) kits from Promega (A-F; bottom panels). Mycoplasma (indicated by solid arrow), mouse (indicated by dashed arrow) and rat (indicated by asterisk) markers are detected in contaminated samples (C-F; top panels) using the "3-in-1" systems - a unique feature which currently used kits (e.g. PPX16HS and GP10) do not have. Overlaid comparison between clean and contaminated samples is shown in G-J. Non-specific peaks generated by human primers on rat DNA are indicated by solid circle. The gender marker, Amelogenin (indicated by arrow head), is the first human marker to appear on the Bioanalyzer electropherograms.

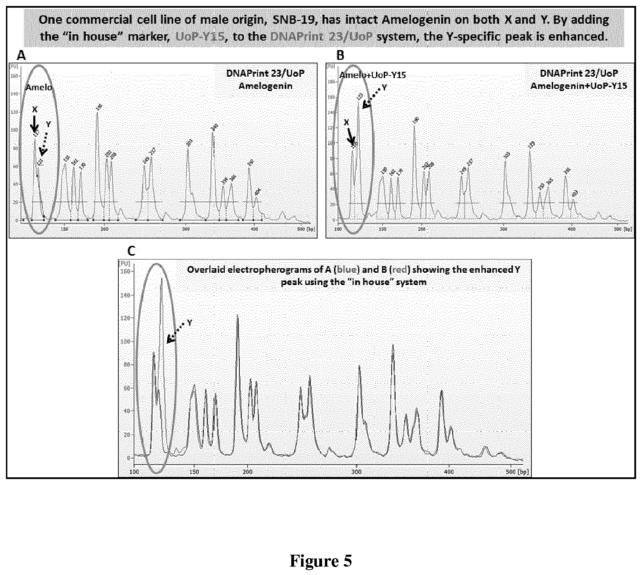

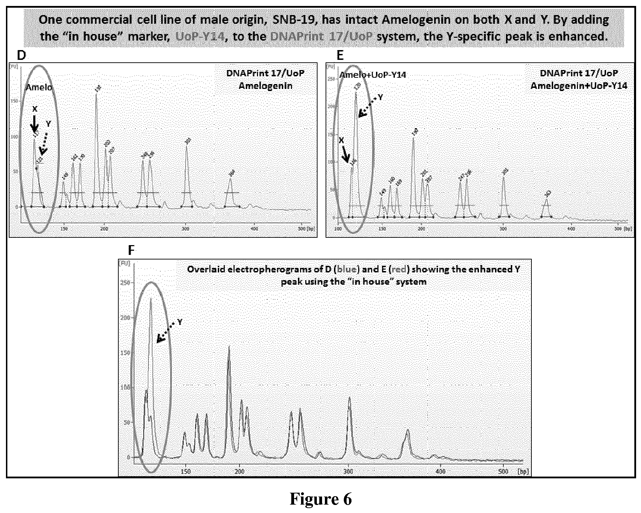

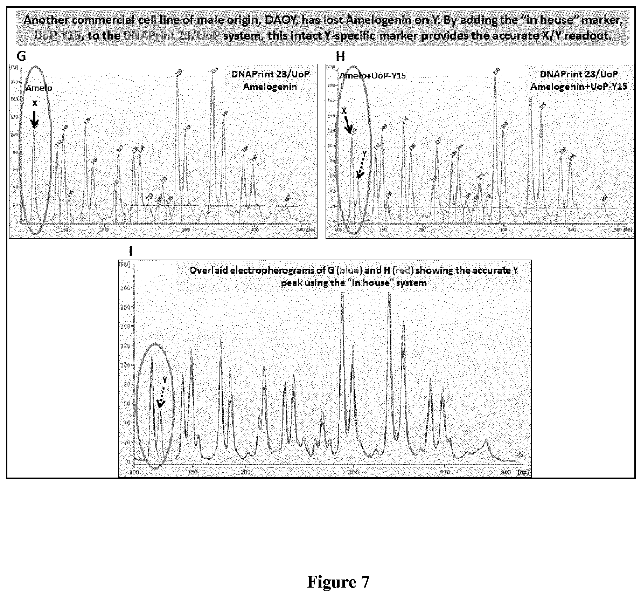

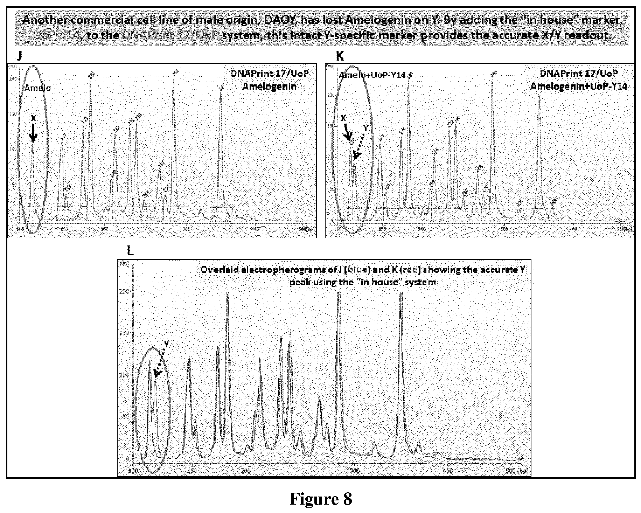

Figure 5: Improved gender determination accuracy by combining Amelogenin and the novel Y-specific markers. Two commercial cell lines of male origin, SNB-19 (maintaining an intact Amelogenin on chromosome Y) and DAOY (missing Amelogenin on chromosome Y), yielded the correct (i.e. X/Y; A & D) and incorrect (i.e. X/X; G & J) readout respectively using the existing gender marker Amelogenin alone. By adding the UoP-Y markers to the DNAPrint 23/UoP and DNAPrint 17/UoP systems, enhanced Y-specific peak was detected in the SNB-19 samples using UoP-Y15 (B) and UoP-Y14 (E) as examples; whereas the accurate readout was obtained in the DAOY samples as illustrated by UoP-Y15 (H) and UoP-Y14 (K). Overlaid electropherograms are shown to highlight the advantage of utilising the "in house" UoP-Y markers (C, F, I & L).

The Agilent 2100 Bioanalyzer offers a broad range of pre-validated analysis kits, including the DNA 1000 chip that has been employed in a previous study (19). This DNA chip provides 5-25bp sizing resolution for fragments of 100-500bp (29). The size range of the commonly used STR markers for human cell identification test is between 100 to 500bp (7, 8, 30); therefore it has been speculated that those amplified STR fragments would be separated on the DNA 1000 chip and produce unique DNA profiles of the cell lines. The PCR amplicons of the mycoplasma, mouse and rat markers in the systems will typically be of 70/1062bp, 90bp, and 500bp sizes respectively in order to differentiate them from the human STR markers.

The new PCR kits are to be used for cell line identification tests. Both kits typically contain 5xEnzyme Mix, 10×Primer Mix, 5×Buffer and PCR-grade H₂O (Fisher; 11506281). The 5xBuffer may contain 250mM KCl, 50mM Tris-HCl (pH 8.3), 0.5% Triton X-100 and 0.8mg/ml BSA. The 5xEnzyme Mix typically consists of the GoTaq® G2 Hot Start Polymerase (Promega, #M740B), magnesium chloride solution (Promega, # A351H) and dNTP (Fisher, #11437120). The 10×Primer Mix typically contains the forward and reverse primers of human, mouse, rat and mycoplasma markers (synthesised and HPLC-purified by Eurofins MWG). In the system, the human-specific STR markers may include the ASN-0002 loci (D5S818, D7S820, D13S317, D16S539, CSF1PO, TH01, TPOX, vWA and Amelogenin) and D21S11. These STR loci collectively provide a DNA profile of the tested cell line with a random match probability of 3.42×10⁻¹⁰ (7). In an expanded (i.e. larger-panel) system, the human-specific STR markers include all those in the above system, plus D3S1358, D8S1179, D18S51, FGA, Penta D and Penta E. These STR loci collectively provide a DNA profile of the tested cell line with a random match probability of 7.09-54.6×10⁻¹⁹ (8). Both systems may also include the "in house" chromosome-Y-specific markers, for example, DYS392-UoP or two or more of the UoP-Y markers (listed in Table 1) for improved gender determination.

In addition, both systems may contain the same rodent and mycoplasma markers whose primer sequences are described above. The PCR primers of two mouse markers amplify a 90bp DNA fragment of mouse-specific sequence on chromosome 4. The PCR primers of one rat marker amplify a 500bp DNA fragment of rat-specific sequence on chromosome 4. The mouse-specific primers bind to the major urinary protein 7 isoform X1 gene on chromosome 4 (NCBI Genbank accession No. NC_000070.6); the rat-specific primers binds to the GTP-binding protein Rheb precursor gene on chromosome 4 (NCBI Genbank accession No. AC_000072.1). The PCR primers of two mycoplasma markers can detect a 70bp and a 1062bp mycoplasma-specific sequences of the 16S ribosomal RNA (rRNA) gene of the eight most common mycoplasma contaminants, *Mycoplasma arginini*, *Mycoplasma fermentans*, *Mycoplasma hominis*, *Mycoplasma hyorhinis*, *Mycoplasma pirum*, *Mycoplasma orale*, *Mycoplasma salivarium* and *Acholeplasma laidlawii.* Both rodent and mycoplasma markers utilised in this invention are species-specific and their PCR primers do not amplify human genomic sequences in order to avoid false positive results caused by non-specific amplification.

The components of the kits are listed in Table 4. Sequences of the primers and estimated fragment sizes are listed in Tables 1-3 (above).

**Table 4. Product components of the Systems (for 50 reactions at 25µl per reaction)**

| Products | Components | |
|---|---|---|
| System 1 | 1 × 250µl | 5×Enzyme Mix**^{a}** |
| "DNAPrint 17" | 1 × 125µl | 10×Primer Mix**^{b}** |
| | 1 × 250µl | 5×Buffer**^{c}** |
| | 2 × 1000µl | PCR-grade H₂O |
| | 1 × 50µl | mouse/rat positive control**^{d}** |
| | 1 × 50µl | mycoplasma positive control**^{e}** |
| System 2 | 1 × 250µl | 5×Enzyme Mix**^{f}** |
| "DNAPrint 23" | 1 × 125µl | 10× 23 Primer Mix**^{g}** |
| | 1 × 250µl | 5×Buffer |
| | 2 × 1000µl | PCR-grade H₂O |
| | 1 × 50µl | mouse/rat positive control |
| | 1 × 50µl | mycoplasma positive control |

| | | |
|---|---|---|
| *^{a} 5*×*Enzyme Mix: 1.0U*/*µl of GoTaq*® *G2 Hot Start Polymerase (Promega, #M740B); 7.5mM of magnesium chloride solution (Promega, # A351H); 1mM of dNTP (Fisher, #11437120)* *^{b} 10×Primer Mix (17 markers in total): **0.8µM** of Amelogenin, D5S818, vWA, D21S11, TPOX, D16S539, CSF1PO; **2µM** of TH01, D13S317, D7S820: 2 mouse markers, 2 mouse markers, 1 rat marker, 2 mycoplasma markers, 2 Y-specific markers;* *^{c} 5*×*Buffer: 250mM KCl, 50mM Tris-HCl (pH8.3), 0.5% TritonX-100 and 0.8mg*/*ml BSA.* *^{d} Mouse*/*rat positive control: 17ng*/*µl of mixed mouse and rat DNAs (1:1; 8.5ng*/*µl each)* *^{e} Mycoplasma positive control: 3pg*/*µl of mycoplasma DNA* *^{f}5xEnzyme mix: 1.2 U*/*µl GoTaq G2 Hot Start Polymerase, 7.5mM magnesium chloride solution; 1mM of dNTP g10*×*Primer Mix (23 markers in total): **0.8µM** of Amelogenin, D5S818, vWA, D21S11, TPOX, D16S539, CSF1PO; **2µM** of D3S1358, TH01, D13S317, D8S1179, D7S820, D18S51, FGA, Penta D, Penta E, 2 mouse markers, 1 rat marker, 2 mycoplasma markers, 2 Y-specific markers* | | |

### PCR set-up and thermal cycling programmes

PCR amplification is set up in a final volume of 25µl reaction, including 1×Enzyme Mix, 1x Primer Mix, 1×Buffer and 10ng of template DNA (see Table 5).

**Table 5. PCR amplification mix for the DNAPrint Systems**

| PCR amplification mix component | Volume/reaction |
|---|---|
| 5×Enzyme Mix | 5µl |
| 10×DNAPrint Primer Mix | 2.5µl |
| 5×Buffer | 5µl |
| H₂O | To a final volume of 25µl |
| DNA (10ng) | Up to 12.5µl |
| Final reaction volume | 25µl |

Three PCR protocols are optimised for use with the "3-in-1" kits of the invention, as shown in Table 6.

**Table 6. Step-by-step protocols for PCR co-amplification of the markers**

| **ABI GeneAmp® PCR System 9600 Thermal Cycler / Bio-Rad MyCycler Thermal Cycler** | **ABI GeneAmp® PCR System 9700 Thermal Cycler** | **ABI Veriti® Thermal Cycler** |
|---|---|---|
| Step 1: 96°C for 3 minutes | Step 1: 96°C for 3 minutes | Step 1: 96°C for 3 minutes |
| Step 2: 94°C for 45 seconds; ramp 68 seconds (0.5°C/s) to 60°C then hold for 60 seconds; ramp 50 seconds (0.2°C/s) to 72°C then hold for 45 seconds ×10 cycles | Step 2: ramp 100% to 94°C for 45 seconds; ramp 29% to 60°C for 60 seconds; ramp 23% to 72°C for 45 seconds ×10 cycles | Step 2: ramp 100% to 94°C for 45 seconds; ramp 22% to 60°C for 60 seconds; ramp 10% to 72°C for 45 seconds ×10 cycles |
| Step 3: 90°C for 45 seconds; ramp 60 seconds (0.5°C/s) to 60°C then hold for 60 seconds; ramp 50 seconds (0.2°C/s) to 72°C then hold for 45 seconds ×22 cycles | Step 3: ramp 100% to 90°C for 45 seconds; ramp 29% to 60°C for 60 seconds; ramp 23% to 72°C for 45 seconds ×22 cycles | Step 3: ramp 100% to 90°C for 45 seconds; ramp 22% to 60°C for 60 seconds; ramp 10% to 72°C for 45 seconds ×22 cycles |
| Step 4: 60°C for 30 minutes | Step 4: 60°C for 30 minutes | Step 4: 60°C for 30 minutes |
| Step 5: 4°C soak | Step 5: 4°C soak | Step 5: 4°C soak |

### FLA by the Bioanalyzer

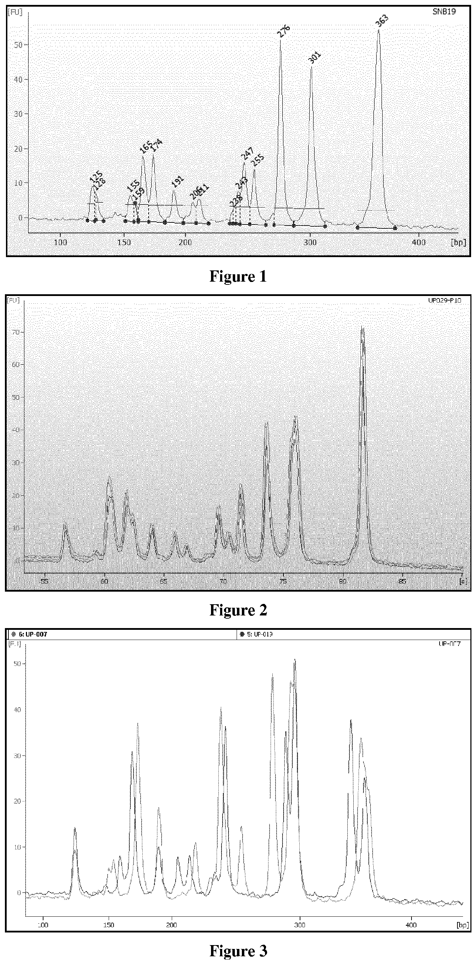

Amplified alleles in the STR-PCR products are separated on the Agilent 2100 Bioanalyzer with the Agilent DNA 1000 Kit, according to the standard protocol with slight modifications (29). In essence, the DNA chip is first primed with 9µl of the gel-dye mixture then 1- 3µl of the PCR product is added to each of the 12 sample wells along with 3-5µl of the internal marker (29). One microlitre of the Agilent DNA 1000 ladder, used as a sizing standard, is loaded into the ladder well with 5µl of the internal marker. After electrophoresis performed with the DNA 1000 assay, the DNA fragments from each sample are analysed using the manufacturer's software, i.e. 2100 Expert, provided with the instrument; the size of each fragment is determined based on the ladder and the internal standards. In addition, the STR markers are identified based on their size ranges (7, 8) and the STR profile of each sample is presented by the sizes and allele numbers of the markers (see example in Table 7; data obtained using the commercial GenePrint® 10 System for the glioma cell line SNB-19), as well as the electropherogram (as represented by SNB-19 in Figure 1).

**Table 7. STR profile of the SNB-19 cell line.**

| FLA | Amelogenin | D5S818 | vWA | TH01 | D13S317 | D21S11 | D7S820 | TPOX | D16S539 | CSF1PO |
|---|---|---|---|---|---|---|---|---|---|---|
| Size (bp) Allele No. | 104, 109 X, Y | 128, 132 11,12 | 146, 154 16,18 | 175, 175 9.3, 9.3 | 182, 186 10,11 | 219,219 29, 29 | 227, 235 10, 12 | 269, 269 8, 8 | 289, 289 12, 12 | 337, 341 11, 12 |

In addition, the comparison analysis featured by the Bioanalyzer 2100 Expert software allows a quick fingerprint check for different DNA samples of the same cell line by overlaying DNA profiles from independent Bioanalyzer assays, as demonstrated in Figure 2 that shows well matched profiles of the UP-029 cell line from 5 different genotyping assays. This comparison analysis can also be performed for two different cell lines in order to compare and confirm their unique profiles, as illustrated in Figure 3 showing distinct profiles of the UP-007 and UP-019 cell lines.

Data from our proof-of-concept experiments indicate that the "3-in-1" DNAPrint kits are comparable to the Promega kits (containing the same panels of SRT markers) in the application of human cell STR profiling, as represented by the commercial human lung cancer cell line, NCI-H1299, in Figure 4A & B. However, our "3-in-1" systems can detect non-human species contamination (mainly by mouse/rat and mycoplasma) using additional specific markers designed under this invention, as illustrated in Figure 4C-F. A comparison between two DNA samples extracted from clean and contaminated NCI-H1299 cells is illustrated by overlaying two electropherograms in Figure 4G-J. Furthermore, our data also suggests that the novel UoP-Y markers can improve upon the existing gender determination marker (i.e. Amelogenin), as demonstrated in Figure 5C, F, I & L.

### References

1. Capes-Davis A, Theodosopoulos G, Atkin I, et al. Check your cultures! A list of cross-contaminated or misidentified cell lines. Int J Cancer. 2010;127:1-8.
2. Masters JR, Thomson JA, Daly-Burns B, et al. Short tandem repeat profiling provides an international reference standard for human cell lines. Proc Natl Acad Sci U S A. 2001;98:8012-8017.
3. Barallon R, Bauer SR, Butler J, et al. Recommendation of short tandem repeat profiling for authenticating human cell lines, stem cells, and tissues. In Vitro Cell Dev Biol Anim. 2010;46:727-732.
4. Nims RW, Sykes G, Cottrill K, Ikonomi P, Elmore E. Short tandem repeat profiling: part of an overall strategy for reducing the frequency of cell misidentification. In Vitro Cell Dev Biol Anim. 2010;46:811-819.
5. American Type Culture Collection Standards Development Organization, ASN-0002 Workshop. Cell line misidentification: the beginning of the end. Nat Rev Cancer. 2010;10:441-448.
6. ANSI/ATCC ASN-0002-2011. Authentication of human cell lines: Standardization of STR profiling. ANSI eStandards Store, 2012.
7. GenePrint® 10 System, Promega Corporation, Technical Manual, Publication Number TM392, 2013.
8. PowerPlex® 16 HS System, Promega Corporation, Technical Manual, Publication Number TMD022, 2012.
9. Butler JM. Genetics and genomics of core short tandem repeat loci used in human identity testing. J Forensic Sci. 2006;51:253-265.
10. Panaro NJ, Yuen PK, Sakazume T, Fortina P, Kricka LJ, Wilding P. Evaluation of DNA fragment sizing and quantification by the agilent 2100 bioanalyzer. Clin Chem. 2000;46:1851-1853.
11. Isaksson HS, Nilsson TK. Preanalytical aspects of quantitative TaqMan real-time RT-PCR: applications for TF and VEGF mRNA quantification. Clin Biochem. 2006;39:373-377.
12. Kuschel M, Neumann T, Barthmaier P, Kratzmeier M. Use of lab-on-a-chip technology for protein sizing and quantitation. JBiomol Tech. 2002;13:172-178.
13. Kataoka M, Fukura Y, Shinohara Y, Baba Y. Analysis of mitochondrial membrane potential in the cells by microchip flow cytometry. Electrophoresis. 2005;26:3025-3031.
14. Miller CL, Diglisic S, Leister F, Webster M, Yolken RH. Evaluating RNA status for RT-PCR in extracts of postmortem human brain tissue. Biotechniques. 2004;36:628-633.
15. Grissom SF, Lobenhofer EK, Tucker CJ. A qualitative assessment of direct-labeled cDNA products prior to microarray analysis. BMC Genomics. 2005;6:36.
16. Becker C, Hammerle-Fickinger A, Riedmaier I, Pfaffl MW. mRNA and microRNA quality control for RT-qPCR analysis. Methods. 2010;50:237-243.
17. Aboud MJ, Gassmann M, McCord BR. The development of mini pentameric STR loci for rapid analysis of forensic DNA samples on a microfluidic system. Electrophoresis. 2010;31:2672-2679.
18. Formosa R, Ravi H, Happe S, et al. DNA-based fish species identification protocol. J Vis Exp. 2010. doi: 10.3791/1871.
19. An Q, Fillmore HL, Vouri M, Pilkington GJ. Brain tumour cell line authentication, an efficient alternative to capillary electrophoresis by using a microfluidics-based system. Neuro-oncology. 2014;16:265-273.
20. Razin S, Yogev D, Naot Y. Molecular biology and pathogenicity of mycoplasmas. Microbiol Mol Biol Rev. 1998;62:1094-1156.
21. Nikfariam L, Farzaneh P. Prevention and detection of Mycoplasma contamination in cell culture. Cell J. 2012;13:203-212.
22. Uphoff CC, Drexler HG. Comparative PCR analysis for detection of mycoplasma infections in continuous cell lines. In Vitro Cell Dev Biol Anim. 2002;38:79-85.
23. Uphoff CC, Drexler HG. Elimination of mycoplasmas from infected cell lines using antibiotics. Methods Mol Biol. 2011;731:105-114.
24. Hay RJ. Operator-induced contamination in cell culture systems. Dev Biol Stand. 1991;75:193-204.
25. Barile MF, Hopps HE, Grabowski MW, Riggs DB, DelGiudice RA. The identification and sources of mycoplasmas isolated from contaminated cell cultures. AnnNY Acad Sci. 1973;225:251-264.
26. Volokhov DV, Graham LJ, Brorson KA, Chizhikov VE. Mycoplasma testing of cell substrates and biologics: Review of alternative non-microbiological techniques. Mol Cell Probes. 2011;25:69-77.
27. Vauhkonen H, Hedman M, Vauhkonen M, Sipponen P, Sajantila A. Typing of XY (male) genotype from malignant neoplastic tissue by the amelogenin-based sex test. J Forensic Sci. 2004;49:222-226.
28. Krenke BE, Tereba A, Anderson SJ, Buel E, Culhane S, Finis CJ, Tomsey CS, Zachetti JM, Masibay A, Rabbach DR, Amiott EA, Sprecher CJ. Validation of a 16-locus fluorescent multiplex system. J Forensic Sci. 2002;47:773-785.
29. Agilent DNA 1000 Kit, Agilent Technologies, Inc. Manual, Publication Number G2938-90014, edition 08/2006. Available at http://www.chem.agilent.com/library/usermanuals/Public/G2938-90014_KitGuideDNA1000Assay_ebook.pdf. Accessed September 26, 2013.
30. AuthentiFiler™ PCR Amplification Kit, Life Technologies Corporation, User Guide, Publication Number 4479553, 2013.

The invention also provides:
1. A cell line identification assay kit comprising:
   (a) one or more genotyping agents;
   (b) one or more rodent-specific agents;
   (c) one or more mycoplasma-specific markers; and optionally
   (d) one or more gender-specific markers.
2. An assay kit according to 1 adapted for use with microfluidic electrophoresis.
3. An assay kit according to 1 or 2 which is adapted to be used with the agents mixed together in a single sample from a cell line.
4. An assay kit according to 1 to 3, which is adapted for use in a polymerase chain reaction (PCR).
5. An assay kit according to any preceding clause, wherein each agent comprises a polynucleotide primer.
6. An assay kit according to any preceding clause, wherein each agent is capable of producing a different detectable product of a different size, and the size of the products from the rodent-specific agent and mycoplasma-specific agent is selected to not substantially overlap with the size of the products from the or each genotyping agent(s).
7. An assay kit according to 6, wherein the detectable product of the rodent agent(s) and mycoplasma agent(s) are polynucleotides having greater than 500 base pairs or less than 100 base pairs (bp).
8. An assay kit according to any preceding clause, comprising two or more mycoplasma agents.
9. An assay kit according to 8, comprising a primer which is mycoplasma-specific for the 16S ribosomal (rRNA) gene of a mycoplasma.
10. An assay kit according to any preceding clause, wherein the rodent-specific agent(s) comprise a rat-specific agent and a mouse-specific agent, preferably a rat-specific agent and two mouse specific agents.
11. An assay kit according to any preceding clause, wherein the rodent specific agent is a mouse specific agent which binds to the major urinary protein isoform X1 gene on chromosome 4.
12. An assay kit according to any preceding clause, wherein the rodent specific agent is a rat specific agent which binds to the GTP-binding protein Rheb precursor gene on chromosome 4.
13. An assay kit according to any preceding clause, comprising two or more Y chromosome specific agents.
14. An assay kit according to any preceding clause, comprising an Amelogenin-specific agent.
15. An assay kit according to 14, additionally comprising a gender-specific agent for a part of the region 0 to 4Mb, 12 to 18Mb, or 20 to 28Mb of the Y chromosome.
16. An assay kit according to any preceding clause, additionally comprising a rodent-specific control and a mycoplasma-specific control.
17. An assay kit according to any preceding clause, wherein the DNA genotyping markers detected by the genotyping agents are short tandem repeats (STR).
18. An assay kit according to 17, wherein the STR markers comprise markers for D5S818, D7S820, D13S317, D16S539, D21S11, CSF1PO, TH01, TPOX, and vWA.
19. An assay kit according to 18, additionally comprising one or more markers selected from, D3S1385, D8S1179, D18S51, FGA, Penta D and Penta E.
20. An assay kit according to any preceding clause, adapted for use with capillary electrophoresis or microfluidic electrophoresis.
21. A method of assaying a cell line, comprising providing a sample of the cell lines and testing the sample for the presence of:
   (a) one or more genotyping markers;
   (b) one or more rodent-specific markers;
   (c) one or more mycoplasma-specific markers; and optionally
   (d) one or more gender-specific markers.
22. A method according to 21, the method comprising the use of microfluidic electrophoresis to detect the presence of the markers.
23. A method according to 21 or 27, wherein each of (a), (b), (c) and optionally (d) are tested in the same sample.
24. A method according to 21 to 23, comprising the use of a kit according to claims 1 to 17 to detect the presence or absence of the markers.
25. A machine readable medium storing executable instructions that when executed by a data processing system cause the system to perform a method comprising:
   (i) obtaining data values obtained by testing a cell line for the presence of:
      (a) one or more genotyping markers;
      (b) one or more rodent-specific markers;
      (c) one or more mycoplasma-specific markers; and optionally
      (d) one or more gender specific markers; and
   (ii) comparing the data to a predetermined value corresponding to the presence of each marker in a cell line; and
   (iii) providing a readout showing the presence or absence of each marker in the cell line.
26. A method according to 25, wherein the data values are obtained by a method according to 21 to 24.
27. A capillary electrophoresis apparatus or microfluidic electrophoresis apparatus, comprising a machine readable medium according to 23 or 24 and/or an assay kit according to claims 1 to 19.

## Claims

1. A cell line identification assay kit comprising:
(a) a plurality of short tandem repeat (STR) loci DNA genotyping agents;
(b) one or more rodent-specific agents;
(c) one or more mycoplasma-specific agents; and
(d) one or more gender-specific agents;
adapted to be used with the agents (a), (b), (c) and (d) mixed together in a single sample from a cell line in a polymerase chain reaction (PCR), and wherein each agent comprises a polynucleotide which is capable of producing a different detectable product of a different size.

2. An assay kit according to claim 1 adapted for use with microfluidic electrophoresis or capillary electrophoresis.

3. An assay kit according to any preceding claims, wherein the detectable product of the rodent agent(s) and mycoplasma agent(s) are polynucleotides having greater than 500 base pairs or less than 100 base pairs (bp).

4. An assay kit according to any preceding claim, comprising two or more mycoplasma agents, preferably comprising a primer which is mycoplasma-specific for the 16S ribosomal (rRNA) gene of a mycoplasma.

5. An assay kit according to any preceding claim, wherein the rodent-specific agent(s) comprise a rat-specific agent and a mouse-specific agent, preferably a rat-specific agent and two mouse specific agents.

6. An assay kit according to any preceding claim, wherein the rodent specific agent is a mouse specific agent which binds to the major urinary protein isoform X1 gene on chromosome 4, or wherein the rodent specific agent is a rat specific agent which binds to the GTP-binding protein Rheb precursor gene on chromosome 4.

7. An assay kit according to any preceding claim, comprising two or more Y chromosome specific agents.

8. An assay kit according to any preceding claim, comprising an Amelogenin-specific agent, preferably additionally comprising a gender-specific agent for a part of the region 0 to 4Mb, 12 to 18Mb, or 20 to 28Mb of the Y chromosome.

9. An assay kit according to any preceding claim, additionally comprising a rodent-specific control and a mycoplasma-specific control.

10. An assay kit according to any preceding claim, wherein the STR markers comprise markers for D5S818, D7S820, D13S317, D16S539, D21S11, CSF1PO, TH01, TPOX, and vWA, more preferably additionally comprising one or more markers selected from, D3S1385, D8S1179, D18S51, FGA, Penta D and Penta E.

11. A method of assaying a cell line, comprising providing a sample of the cell lines and testing the sample using polymerase chain reaction (PCR) for the presence of:
(a) a plurality of short tandem repeat (STR) loci DNA genotyping markers;
(b) one or more rodent-specific markers;
(c) one or more mycoplasma-specific markers; and
(d) one or more gender-specific markers,
wherein each of (a), (b), (c) and optionally (d) are tested in the same sample and each agent comprises a polynucleotide primer which produces a different detectable product of a different size.

12. A method according to claim 110, the method comprising the use of a microfluidic electrophoresis or capillary electrophoresis to detect the presence of the markers.

13. A machine readable medium storing executable instructions that when executed by a data processing system cause the system to perform a method comprising:
(i) obtaining data values obtained by testing a cell line using a kit according to claims 1 to 10, for the presence of:
(a) a pluarality of short tandem repeat (STR) DNA genotyping markers;
(b) one or more rodent-specific markers;
(c) one or more mycoplasma-specific markers; and
(d) one or more gender specific markers; and
(ii) comparing the data to a predetermined value corresponding to the presence of each marker in a cell line; and
(iii) providing a readout showing the presence or absence of each marker in the cell line.

14. A method according to claim 13, wherein the data values are obtained by a method according to claims 11 to 12.

15. A capillary electrophoresis apparatus or microfluidic electrophoresis apparatus, comprising a machine readable medium according to claim 14.
